# EUROPEAN PATENT SPECIFICATION

(11) **EP 2 540 243 B1**
(45) Date of publication and mention of the grant of the patent: **26.02.2020**
(21) Application number: 11171770.8
(22) Date of filing: 28.06.2011
(51) Int. Cl.: A61B 18/12, A61B 18/14, A61B 18/00

(54) **An electrosurgical instrument**
Elektrochirurgisches instrument
Instrument électrochirurgical

(43) Date of publication of application: 02.01.2013
(73) Proprietor: Lina Medical ApS, 2600 Glostrup (DK)
(72) Inventor: Poulsen, Henrik Bisgaard, 3550 Slangerup (DK)
(74) Representative: Inspicos P/S

(56) References cited:
- EP-A1- 2 210 567
- WO-A1-98/07378
- WO-A1-2010/092328
- WO-A2-2005/110263
- WO-A2-2008/071914

## Description

The present invention relates to a method of operating an electrosurgical instrument connected to an electrosurgical generator and comprising a circuit, a measurement unit and at least one electrode, where the circuit controls a switching of the electrosurgical instrument between a measurement mode, where the measurement unit measures the impedance between the electrodes, and an operating mode, where the electrosurgical generator applies a voltage over the electrodes.

Within the context of the present application the term "bipolar electrosurgical instrument" is to be understood as an electrosurgical instrument where both electrodes are part of the electrosurgical instrument itself. The flow of electricity is substantially confined to the space between the opposite electrode faces of the electrosurgical instrument, such as e.g. bipolar forceps or bipolar loop electrodes, so that tissue damage is substantially confined to tissue situated between said electrodes. Since no dispersive electrode is required remote from the patient, less electrical current is needed to obtain the same effects than when using monopolar electrosurgical instruments, where the current must pass through the body to get to the dispersive electrode.

Within the context of the present application the term "bipolar electrosurgical generator" is synonymous with a high frequency generator, in particular a radio frequency generator, for use with a bipolar electrosurgical instrument. A "bipolar electrosurgical generator" may thus also be a multipurpose electrosurgical generator with both monopolar and bipolar functions. Within the context of the present application bipolar or monopolar electrosurgical generators and bipolar or monopolar electrosurgical instruments can be used.

Although bipolar electrosurgical procedures in general are seen as being safer than monopolar electrosurgical procedures there are still a need for improvements. Some of the above disadvantages and risks of causing injuries when using bipolar electrosurgical instruments are attempted remedied by incorporating technology that senses tissue resistance and automatically are able to adjust voltage in relation to a predetermined threshold value to compensate for different kinds of tissue and are also able to switch off the voltage if the health of the patient is at risk. However, most electrosurgical generators are customised for use with a limited number of electrosurgical instruments. Other electrosurgical generator models cannot be used instead, and hospitals and medical/surgical performers are compelled to buy new and/or additional equipment to benefit from the advantages of developing technology within electrosurgical instruments.

For surgery the surgeon presets the bipolar electrosurgical generator at a selected power output level based on guidelines made by the provider, as well as other indications such as previous experiences and other kinds of safety regulations. When during the surgical procedure, the electrodes have cut through the tissue object positioned between the electrodes it is important from a security point of view for the patient that the power from the generator to the electrodes are shut down as fast as possible to avoid that by accident other tissue are burnt or cut.

It is realised that there remains a need within the art to develop cost-saving electrosurgical techniques and instruments towards eliminating unintended effects and limitations related to their uses.

It is a main aspect of the present invention to provide an electrosurgical instrument that is safer than known instruments.

In a second aspect according to the present invention is provided an inexpensive and flexible bipolar electrosurgical instrument.

In a third aspect according to the present invention is provided a bipolar electrosurgical instrument with coated electrodes that perform better than known instruments with coated electrodes, in particular with Teflon® coated electrodes.

In a fourth aspect according to the present invention is provided a bipolar electrosurgical instrument.

The novel and unique whereby these and other aspects are achieved according to the present invention consists in the fact that the circuit comprises a timer that periodically controls the switching of the electrosurgical instrument between the measurement mode and the operating mode.

Conventional electrosurgical generators can be utilized with the instrument according to the present invention. The electrosurgical generator may be configured to operate in e.g. cut mode, blend mode, and/or coagulation mode, e.g. using the electrosurgical instruments disclosed in the applicants own international patent application no. WO 2008/010150, and European patent applications no. EP 1 089 664 and EP 1 150 616.

The input end of the electrosurgical instrument may e.g. be electrically connected to the electrosurgical generator by means of more or less long electrical wires and plugs, e.g. a power cable with euro connector.

That the impedance can be measured when the cutting and coagulating RF-frequency signal is shut off means that the measurement unit does not need to be constructed for high voltage, which means an economical benefit. The idea according to the present invention enables a DC signal to be applied by and the current to be measured by the measurement unit.

In a preferred embodiment the electrosurgical instrument can comprise at least two electrodes.

Contemplated electrosurgical instrument according to the present invention includes but are not limited to electrosurgical instruments configured to provide at least one of the functions cut, coagulate, and ablate. In particular the electrosurgical instrument can be a bipolar forceps, bipolar loop electrode, bipolar scissors, bipolar pencil, bipolar scalpel, or a bipolar electrosurgical instrument incorporating and combining one or more functions thereof. The electrosurgical instrument can also be a monopolar pencil or scalpel. Optionally at least one of the electrodes of the electrosurgical instrument has an area provided with a non-stick coating.

The electrodes in the measurement mode can be identical to the electrodes in the operating mode.

When the measurement unit in the measurement mode registers that the impedance is zero or nearly zero (below another preset value) the timer will be stopped to switch over the electrosurgical instrument to the operating mode. In this way the electrodes will not be destroyed by overheating. Very hot elements can also be a danger for surrounding tissue.

In a preferred embodiment the timer can periodically control the switching of the electrosurgical instrument between the measurement mode and the operating mode with a period that is preferably between 0.05 and 0.15 of the period in the measurement mode and 0.85 and 0.95 of the period in the operating mode and most preferably one tenth of the period in the measurement mode and nine tenth of the period in the operating mode.

When the instrument is in the measurement mode no cutting or coagulation occurs. A period comprises the operating mode and the measurement mode. When the share of the measurement mode increases at the expense of the operating mode the power distributed to the tissue will decrease. For that reason it can be an advantage to have as high ratio between the time of the operating mode and the time of the measurement mode as possible. From the other point of view the measurement mode must be long enough to get a reliable value of the impedance. If the period time is long the ratio between the time of the operating mode and the time of the measurement mode can be higher while when the period time is short the ratio must be lower. A value of the ratio between 5.6 (0.85 divided by 0.15) and 19 (0.95 divided by 0.05) is suitable and most preferably the ratio should be 9. But the ratio can also be as high as 99 especially if the period time is relatively long.

In an expedient embodiment according to the present invention, the timer can periodically control the switching of the electrosurgical instrument between the measurement mode and the operating mode with a period that is less than 5 sec. long, preferably less than 1 sec. long, more preferably less than 300 msec. long, even more preferably less than 100 msec. long, yet more preferably less than 30 msec. long, yet even more preferably less than 10 msec. long, further more preferably less than 3 msec. long, even further more preferably less than 1 msec. long, yet further more preferably less than 300 µsec. long, yet even further more preferably less than 100 µsec. long and most preferably less than 30 µsec. long.

Using the short period the energy driven through the electrodes and the tissue will be very small after the measurement unit has measured that the impedance is above a preset value or below another preset value. By using a short period the electrosurgical instrument will very fast stay in the measurement mode.

In another embodiment an at least one first switch, controlled by the circuit, when going from the operating mode to the measurement mode, can disconnect the electrosurgical generator from the electrodes and can connect the measurement unit to the electrodes.

It can be important that the at least one first switch can disconnect the electrosurgical generator from the electrodes very fast. The switch can also be able to disconnect the signal from the electrosurgical generator even though the voltage and the current are high.

The circuit comprising the timer can optionally be integrated in the electrosurgical instrument in a manner that allows it to be switched on or off during surgery. Circuits with different relative proportions of the period time for the measurement mode and the operating mode and circuits with different total period times can be integrated in a electrosurgical instrument and be exchanged if necessary. The circuit can comprise integrated circuits, which are possible to control by a computer. By connecting a computer to the circuit and running a suitable software it can be possible to change the properties of the integrated circuits and then change the relative periodic times between the measurement mode and the operating mode and also change the time of the period.

In yet another embodiment an at least one second switch, controlled by the circuit, when going from the measurement mode to the operating mode can disconnect the measurement unit from the electrodes and can connect the electrosurgical generator to the electrodes.

The at least one first and the at least one second switch can be the same switch. The first and the second switch can be of the same type. The first and the second switch can be relays or electronic switching circuits based on semi-conductors. The electronic switching circuits can be diodes, transistors, triacs, Darlington transistors and/or integrated circuit chips. It should be understood that the electronic switching circuits could be any switching circuit known to the person skilled in the art.

In a favourable embodiment the measurement unit in the measurement mode can apply a voltage over the electrodes, measure the current through the electrodes, calculate the impedance of an object between the electrodes and if the impedance is below a first predefined limit or above a second predefined limit the circuit will not switch over to the operating mode.

The benefit of the present invention is for the safety of the patient being treated by the instrument. When the electrodes have come through a tissue object during cutting or coagulating the measurement unit will, when the instrument is in the measurement mode, measure impedance between the electrodes that is below a preset value, and the timer will not switch over to the operating mode but stay in the measurement mode. It is also possible that the impedance will be infinite when the tissue object is cut through because then there is nothing between the two electrodes where the cutting or coagulating current can flow. For that reason, when the measured impedance exceeds a preset value the instrument is not switched over to the operating mode. That the instrument is not switched over to the operating mode means that there will be no high voltage between the electrodes and there is no risk that by accident the electrodes cut, burn or damage any tissue that should not be treated. The surgeon can safely remove the instrument. Especially if the surgery has been performed in a body cavity where there is not so much space there is a risk, when the electrodes have just dissected the tissue object, that the surgeon is surprised and is not able to react before the surgeon looses the grip at the instrument and drops the instrument in the cavity. If the surgeon is not fast enough to compensate for the falling down of the instrument and the high voltage from the electrosurgical generator is still applied over the electrodes the electrodes will damage tissue that should not be damaged. In a worst scenario a big blood vessel is cut over and blood is being sprayed all over and the patient's life can be in danger. That the instrument all the time and with a relatively high frequency switches from the measurement mode to the operating mode enables the instrument to very fast stop the power feeding to the electrodes when the tissue object between the electrodes are cut over or coagulated.

In a useful embodiment the measurement unit can calculate a cutting voltage or a coagulating voltage based on the impedance and that the measurement unit can control the electrosurgical generator to apply the calculated cutting voltage or coagulating voltage in the operating mode.

The invention can also relate to an electrosurgical instrument comprising a circuit, a measurement unit and at least one electrode, where the electrosurgical instrument has means to be connected to a electrosurgical generator and where the circuit has means to switch the electrosurgical instrument between a measurement mode, where the measurement unit measures the impedance between the electrodes, and an operating mode, where the electrosurgical generator applies a voltage over the electrodes, where the circuit comprises a timer that periodically controls the switching of the electrosurgical instrument between the measurement mode and the operating mode.

Advantageously, the electrosurgical instrument can comprise at least two electrodes.

The circuit can comprise the measurement unit. The circuit can also comprise means in the form of a timer to give a signal to switch the electrosurgical instrument from the operating mode to the measurement mode. The signal can be a voltage over an electromagnet that closes and/or opens switches. In the operating mode the switches that can connect the electrodes with the electrosurgical generator is closed and the switches that can connect the measurement unit to the electrode, to measure the impedance of the object between the electrodes, are open. In the measurement mode the switches can be positioned contrary so that the electrosurgical generator can be shut off from the electrodes and the measurement unit can be connected to the electrodes. Instead of using electromagnetically controlled switches the switches can be semi-conductor based. Switching means built up of semi-conductor elements will be much faster and more accurate than electromagnetically controlled switches.

In a preferred embodiment the circuit can have at least one first switch and at least one second switch, controlled by the circuit, where in the measurement mode the at least one first switch can be in a mode so that the electrosurgical generator is disconnected from the electrodes and the at least one second switch can be in a mode so that the measurement unit is connected to the electrodes.

The electrosurgical generator can be disconnected from the measurement unit and from the electrodes during measurement of current through the tissue between and in electrical contact to the electrodes when a voltage is applied over the electrodes by e.g. the measurement unit. The advantage of disconnecting the electrosurgical generator in this case is that the measurement unit does not need to be built for high voltage but only for low voltage, which will make the construction of the measurement unit much cheaper.

In an advantageous embodiment the circuit can have at least one third switch and at least one fourth switch, controlled by the circuit, where in the operating mode the at least one third switch can be in a mode so that the electrosurgical generator is connected to the electrodes and the at least one fourth switch can be in a mode so that the measurement unit is disconnected from the electrodes.

Here as well, the advantage is that the measurement unit does not need to be constructed for high voltage, which means an economical benefit.

The at least one third switch can be the at least one first switch and the at least one fourth switch can be the at least one second switch.

The at least one first and the at least one second switch can be the same switch. Two, three or four of the first, second, third and/or fourth switch can be of the same type. The first, second, third and fourth switch can be relays or electronic switching circuits based on semi-conductors. The electronic switching circuits can be diodes, transistors, triacs, Darlington transistors and/or integrated circuit chips. It should be understood that the electronic switching circuits could be any switching circuit known to the person skilled in the art.

In a useful embodiment the measurement unit can have at least one of the following means or combinations thereof: means to apply a voltage over the electrodes, means to measure the current through the electrodes, means to calculate the impedance of an object between the electrodes and can have means to, if the impedance is below a first predefined limit or above a second predefined limit, stop the circuit to switch over the electrosurgical instrument to the operating mode.

The means to apply a voltage can be a voltage source e.g. placed in the measurement unit but could also be placed somewhere else in the electrosurgical instrument. The voltage source can drive a current through the tissue between and in electrical contact to the electrodes. The means to measure the current can be an amperemeter.

In the measurement mode the measurement unit can apply a voltage over the electrodes, measure the current through the tissue between and in electrical contact to the electrodes and calculate the impedance of the tissue between the electrodes. If the calculated impedance is above a certain preset value the electrodes are assumed not to be in contact with the tissue and the timer will not send a signal to switch the electrosurgical instrument over to the operating mode.

A situation where a high voltage is applied over the electrodes without the electrodes being in contact with anything means a danger that the electrodes by accident touches and destroys tissue, that should not be cut, coagulated or removed. Since the electrosurgical instrument according to the present invention is not switched over to the operating mode, when the impedance is too high, that danger is eliminated.

When the measurement unit in the measurement mode registers that the impedance is zero or nearly zero (below another preset value) the timer can be stopped to switch over the electrosurgical instrument to the operating mode. In this way the electrodes will not be destroyed by overheating. Very hot elements can also be a danger for surrounding tissue. If the electrosurgical generator drives current through elements, which are shorted to each other, normally the electrosurgical generator will deliver no current. The measurement unit can have means to tell the user that the elements are shorted to each other. To know why the electrosurgical generator does not deliver any power can be decisive if suddenly an artery has started to bleed very heavily and the bleeding needs to be stopped immediately by coagulation to save the life of the patient.

The invention will be described in further details below with reference to the accompanying drawing illustrating as an example a circuit with a timer and a measurement unit incorporated in an electrosurgical loop electrode instrument.
Fig. 1 shows, in a perspective principle sketch, a bipolar electrosurgical loop instrument coupled to a bipolar electrosurgical generator,
fig. 2a shows the timer/measurement unit controlling the switches, where the switches are relays and where the switches are positioned so that the instrument is in the operating mode so that the power from the bipolar electrosurgical generator is provided to the electrodes,
fig. 2b shows the timer/measurement unit controlling the switches, where the switches are relays and where the switches are positioned so that the instrument is in the measurement mode so that the timer/measurement unit is connected to the electrodes and is able to measure the impedance between the electrodes,
figs. 3a and 3b show the timer/measurement unit controlling the switches, where the switches are electronic relays,
figs. 3c and 3d show two embodiments of the electronic relays,
fig. 4 shows a schematic view of a bipolar electrosurgical loop instrument with an alternative loop electrode,
figs. 5a and b show the holding member and how the holding member connects the ends of the metal wire parts of the loop electrode, and
figs. 6a - d show further alternative loop electrodes.

In the figures a bipolar electrosurgical generator is, as a non-limiting example, shown with the bipolar electrosurgical instrument according to European patent application no. EP09151319 modified with a circuit comprising a timer/measurement unit controlling switches according to the invention. The proportions between timer/measurement unit, bipolar electrosurgical instrument and bipolar electrosurgical generator are not to be taken for real, said proportions are used to better illustrate the components of the bipolar electrosurgical devices according to the present invention.

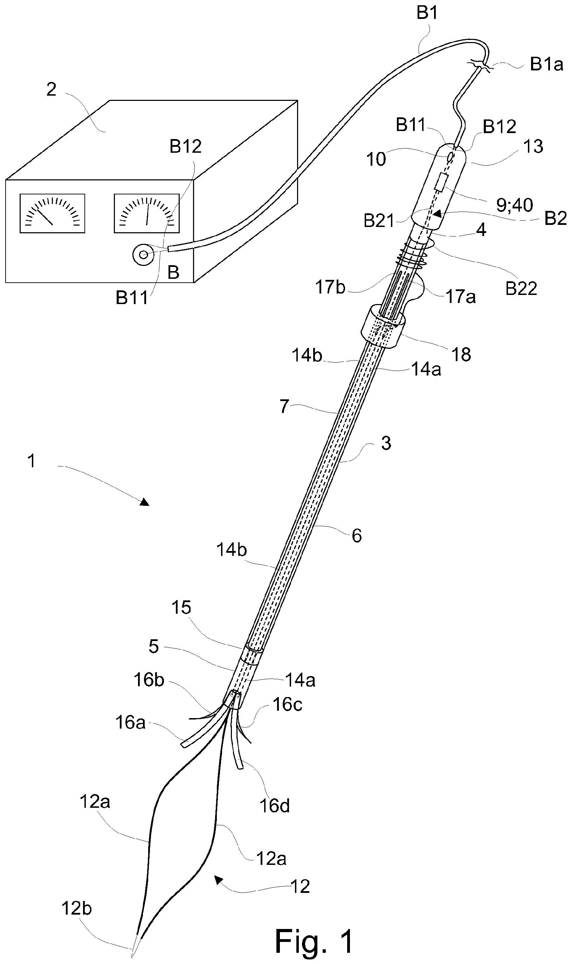

Fig. 1 shows, in perspective, a general view of a bipolar electrosurgical instrument 1 connected to a bipolar electrosurgical generator 2. The bipolar electrosurgical instrument 1 has a hollow shaft 3 with a proximal end 4 and a distal end 5. The hollow shaft 3 is defined at least by an outer tubular casing 6 inside which an inner tubular casing 7 extends reciprocatingly. An output B of the bipolar electrosurgical generator 2 providing alternating current is connected to a circuit 9 via electric wire B1 with electric conductors B11 and B12. In fig. 1 the electric wire B1 is for illustrative purposes shown fragmented by fragment lines B1a.

In one or both of the electric conductors B11 and B12 an actuation knob 10 connects and disconnects the bipolar electrosurgical generator 2 from the circuit 9. Two electric conductors B21 and B22 forming an electric wire B2 connect the circuit 9 to a loop electrode 12, such as an electrically conductive resilient looped wire, via the electric conductors B21. The loop electrode has an electrically isolating layer 12a except at the tip 12b, where the loop electrode is bare. Four electric electrode wires 14a,14b of which only two are visual in fig. 1, extend slidingly lengthwise in the circumferential space between the inner tubular casing 7 and the outer tubular casing 6 in recesses in one or more spacer plugs 15, of which only one can be seen in fig. 1. Each electric electrode wire 14a,14b has a distal end configured as a flat conductive plate or blade 16a,16b,16c,16d of enlarged surface area to establish good contact with an object (not shown) to be cut. The electric electrode wires 14a,14b are isolated from the loop electrode 12. The electric electrode wires 14a,14b have proximal ends connected to the electric conductor B22. The bipolar electrosurgical generator 2 supplies the loop electrode 12 and the four flat conductive plates or blades 16a,16b,16c,16d with electrical power to enable electrosurgical cutting or coagulating between the loop electrode 12 and the four flat conductive plates or blades 16a,16b,16c,16d.

The inner tubular casing 7 accommodates, as indicated by dashed line, the loop electrode 12. The actuation knob 10 is situated on a first handle 13 at the proximal end of the inner tubular casing 7. The actuation knob 10 enables the surgeon to switch cutting or coagulating current through the loop electrode 12, an object (not shown) and the four flat conductive plates or blades 16a,16b,16c,16d on and off on demand to enable electrosurgical cutting or coagulating and according to the surgeons choice only by a simple pressure on the actuation knob 10. The first handle 13, that incorporates the circuit 9 comprising the timer/measurement unit, is also used for reciprocating the loop electrode 12.

The outer tubular casing 6 has at its proximal end 4 a number of longitudinal slide grooves 17a,17b of which only two can be seen in fig. 1. The number of slide grooves 17a,17b corresponds to the number of electric electrode wires 14a,14b. The proximal ends of the electric electrode wires 14a,14b are passed through the corresponding slide grooves 17a,17b and secured to a second handle 18 for reciprocating said blade electrodes 16a, 16b, 16c, 16d in and out of the distal end 5 of the hollow shaft 3 as occasion requires.

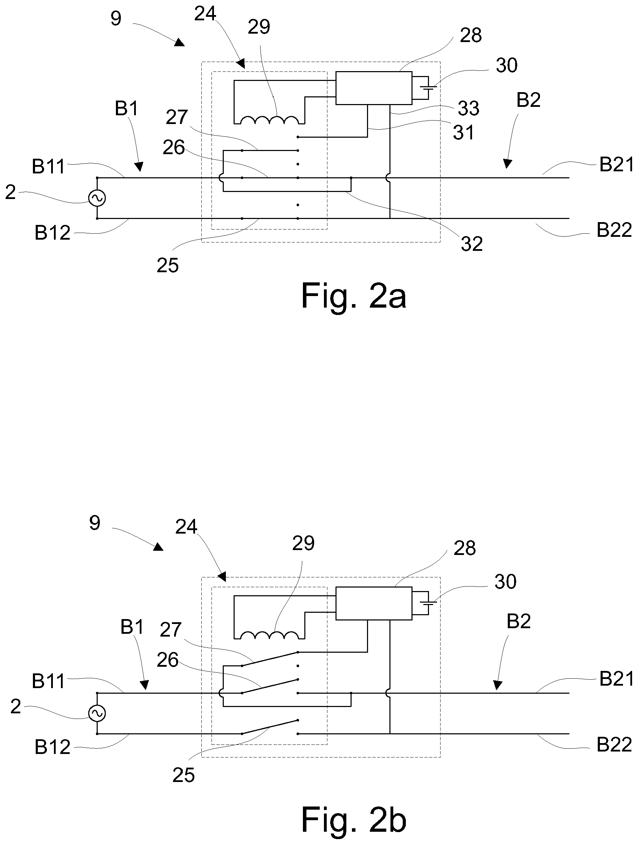

In the exemplary bipolar electrosurgical instrument 1, the circuit 9 comprising the timer/measurement unit, which is shown in the principle sketch of enlarged scale view of fig. 2, is integrated in the first handle 13 and electrically inserted between the electric wires B1 and B2 to enable the surgeon to use another voltage than actually delivered by the bipolar electrosurgical generator 2 to the loop electrode 12.

If the bipolar electrosurgical generator 2 provides an electric current to the bipolar electrosurgical instrument 1, an electric current will flow via one of the conductors of the electric wire B1 through the circuit 9 and via the electric conductor B21 through the loop electrode 12 to the tip 12b over to an object (not shown), one or more of the four flat conductive plates or blades 16a,16b,16c,16d, the corresponding electric electrode wires 14a,14b and the electric conductor B22 back to circuit 9 and the other conductors of the electric wire B1 back to the bipolar electrosurgical generator.

The circuit 9, as shown in fig. 1, is as mentioned configured to be incorporated in the tubular handle 13, or any other part of the electrosurgical instrument 1, where the first handle 13 or other suitable part fits the palm of the hand of the surgeon.

A typical bipolar electrosurgical generator for use in the present invention transmits high-frequency (HF) energy ranging from 350 KHz to 1 MHz.

When an object (not shown) is situated between and in electrical contact with the electrodes, which means in electrical contact with the spread apart four flat conductive plates or blades 16a,16b,16c,16d and also in electrical contact with the tip 12b of the loop electrode 12, it is possible to establish a value of the impedance, resistance or capacity to be used as an indication of the presence of an object to inform the surgeon that cutting or coagulating current can be switched on, or off if no contact is established, or that voltage needs amplification. The impedance of the tissue object to be cut or coagulated is measured and maybe also calculated by the timer/measurement unit.

In figs. 2a and 2b the circuit 9 placed between the electric wires B1 and B2 is shown. The circuit comprises a relay 24, where the relay controls switches 25,26 between the bipolar electrosurgical generator 2 and the electrodes, as well as a switch 27. The relay is controlled by a timer/measurement unit 28 through e.g. an electromagnet 29. The timer/measurement unit 28 might have a battery 30 to be able to operate or are supplied by the bipolar electrosurgical generator 2, e.g. by a low voltage output (not shown) or by the output B. All three switches 25, 26, 27 are changed simultaneously to go from operation mode to measurement mode and back again in a recurring cycle.

When the switches 25, 26 are closed the switch 27 is opened and vice versa. In the operation mode, which can be e.g. 9/10 or 99/100 of the cycle, the switches 25, 26 are closed and the switch 27 is open. In the operation mode the bipolar electrosurgical generator 2 will drive current via the switches 25, 26 through the loop electrode, an object (not shown), the flat conductive plates or blades 16a, 16b, 16c, 16d and back again to the bipolar electrosurgical generator 2. When the e.g. 9/10 or 99/100 of the cycle has elapsed the timer/measurement unit 28 switches the relay to the measurement mode by opening the switches 25, 26 and closing the switch 27. In the measurement mode, which can then be e.g. 1/10 or 1/100 of the cycle, the timer/measurement unit 28 is connected to the electrodes, like e.g. the loop electrode 12 and the flat conductive plates or blades 16a, 16b, 16c, 16d, via conductors 31, 32, 33, B21, B22 and switch 27. By applying a voltage over the electrodes and measuring the current through the electrodes and the object (not shown), the timer/measurement unit 28 has means to calculate the impedance of the object. The timer/measurement unit 28 can have means to calculate the current needed to easily and effectively cut the tissue or coagulate the tissue and avoid driving so much current through the tissue that the tissue will burn too heavy creating too much unhealthy smoke. If the calculated impedance is infinite the change over to the operation mode might be stopped. If the calculated impedance is zero indicating that the two electrodes are in touch with each other the change over to the operation mode might also be stopped, since there is no tissue between the electrodes and/or the electrodes can be damaged by the current.

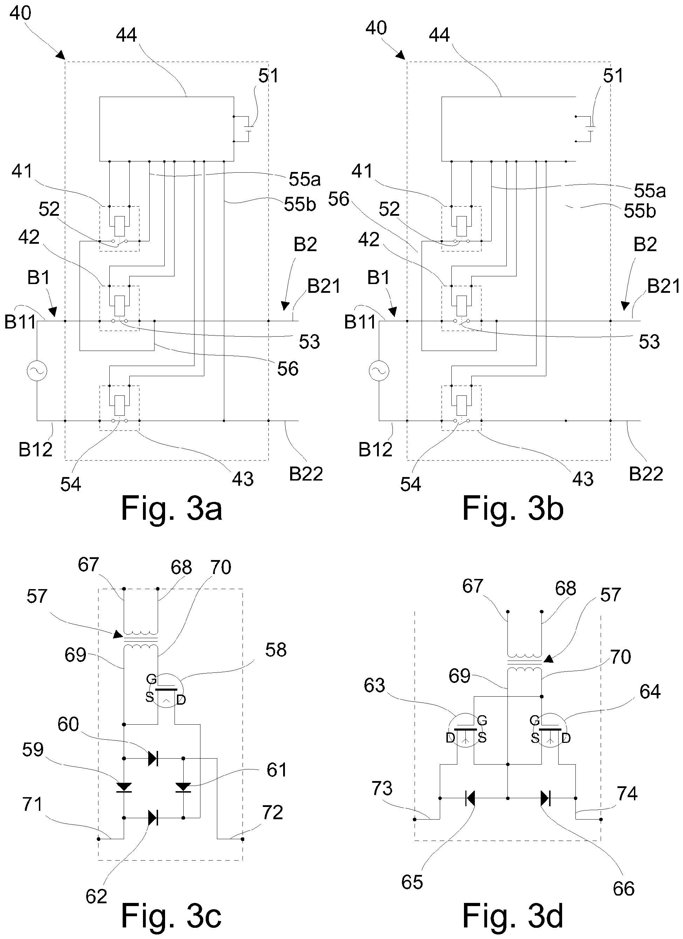

Figs. 3a and 3b disclose like in figs. 2a and 2b a circuit 40 placed between the electric wires B1, comprising electric conductors B11 and B12, and B2, comprising electric conductors B21 and B22. The circuit 40 in figs. 3a and 3b have all the properties and can be described in exactly the same way as the circuit 9 in figs. 2a and 2b, except that the relays in circuit 40 are electronic relays 41, 42, 43. A timer/measurement unit 44 controls the electronic relays. The timer/measurement unit 44 can be driven by a battery 51, by the bipolar electrosurgical generator 2 or by any other power supply. The timer/measurement unit 44 in the operation mode shown in fig. 3a controls the electronic relay 41 to keep a switch 52 open and controls the electronic relays 42 and 43 to keep switches 53 and 54 closed so that the bipolar electrosurgical generator 2 is able to drive a cutting or coagulating current in preferably the following order: through the loop electrode 12, the object (not shown) and the four flat conductive plates or blades 16a, 16b, 16c, 16d.

In the measurement mode, shown in fig. 3b, the timer/measurement unit 44 controls the electronic relays 41, 42, 43 to keep the switches 52, 53, 54 in a position opposite to the position in the operation mode. In the measurement mode, an electrical conductor 55a is connected to an electrical conductor 56 and the timer/measurement unit 44 has means to apply a voltage over the electrodes, measure the current through the object and calculate the impedance of the object.

All three switches 52, 53, 54 are changed simultaneously to go from operation mode to measurement mode and back again in a recurring cycle.

In figs. 3c and 3d two different embodiments of the electronic relays 41, 42, 43 are disclosed. The electronic relay in fig. 3c comprises a pulse transformer 57, a MOSFET transistor 58, and four diodes 59, 60, 61, 62. In fig. 3d the electronic relay comprises the pulse transformer 57, two MOSFET transistors 63, 64 and two diodes 65, 66.

A control voltage in form of a pulse is applied by the timer/measurement unit 44, via connections 67, 68 over the pulse transformer 57. The control voltage over the connections 67, 68 is transferred by the pulse transformer and applied on the connections 69, 70 as a terminal voltage. The transformer 57 can amplify the signal from the timer/measurement unit 54 to the connections 79, 80 but the transformer can also be a 1:1-transformer. The object of the transformer 57 is to isolate the control voltage from the terminal voltage, so that the control voltage and the terminal voltage can be at different potentials without disturbing or interfering with the electronics in the timer/measurement unit 54. The terminal voltage will be applied over the gate G and the source S (V_{GS}) of the MOSFET transistor 58 in fig. 3c and of the two MOSFET transistors 63, 64 in fig. 3d. The terminal voltage exceeds the threshold voltage (V_{GS} > V_{TH}) of the one or more MOSFET transistors and opens for current to flow between the drain D and the source S of the one or two MOSFET transistors.

The pulse transformer is designed to keep the positive charge at the gate long enough to keep the one or two MOSFET transistors open until the circuit 40 changes from operation mode to measurement mode and vice versa.

To close or block the one or two MOSFET transistors and stop current to flow from the drain D to the source S a pulse with a negative potential is sent to the pulse transformer 57 by the timer/measurement unit 44. The pulse with the negative potential is transferred by the pulse transformer and applied over the gate G and the source S so that V_{GS} < V_{TH}.

If V_{GS} > V_{TH} of the MOSFET transistor 58 in fig. 3c is true, the bipolar electrosurgical generator 2 can drive current going into the electronic relay at a connection 71, through diode 62, through the MOSFET transistor 58 from the drain to the source, through the diode 60 and out through a connection 72 and further to the object. The bipolar electrosurgical generator 2 can also drive the current in through the connection 72, then through the diode 61, through the MOSFET transistor 58 from the drain to the source, through the diode 59 and out through the connection 71 back to the bipolar electrosurgical generator 2. Likewise, the electronic relay in fig. 3c can, when the connection 71 is connected to the connection 55a from the timer/measurement unit 44, be used to allow or to block the timer/measurement unit 44 to drive current from the connection 55a through the connections 71, 72, further via connections 56, B21, the object and back through B22 and a connection 55b to the timer/measurement unit 44.

In fig. 3d a connection 73 will connect to the bipolar electrosurgical generator 2 or to the connection 55a from the timer/measurement unit 44. When the terminal voltage is applied over the gate G and the source S of the two MOSFET transistors 63, 64 current entering through connection 73 will pass the MOSFET transistor 63 from drain D to source S and through the diode 66 and out through a connection 74, while a current entering through the connection 74 will pass the MOSFET transistor 64 from drain D to source S and through the diode 65 and out through the connection 73.

The advantage of using the semi-conductor based circuit instead of the circuit 9 disclosed in figs. 2a and 2b is that the semi-conductor based circuit will be able to shift much faster between the operation mode and the measurement mode. With the semi-conductor based circuit it will be possible to shift between the operation mode and the measurement mode and back again in milliseconds and even in microseconds.

Instead of using MOSFET transistors other FET transistors can be used like e.g. IGBT. The channel of the FET can either be doped to produce an N-type semiconductor or a P-type semiconductor. The transistors can also be NPN-transistors or PNP- transistors or the circuit can be made of any kind of semiconductor. Likewise the pulse transformer to control the one or more transistors can also be an Optomos®, any kind of optical relay or any kind of gate driver circuit. The person skilled in the art will know how to design modifications of such a semi-conductor based circuit and such an optical relay or gate driver circuit. Such modifications are also intended within the scope of the present invention.

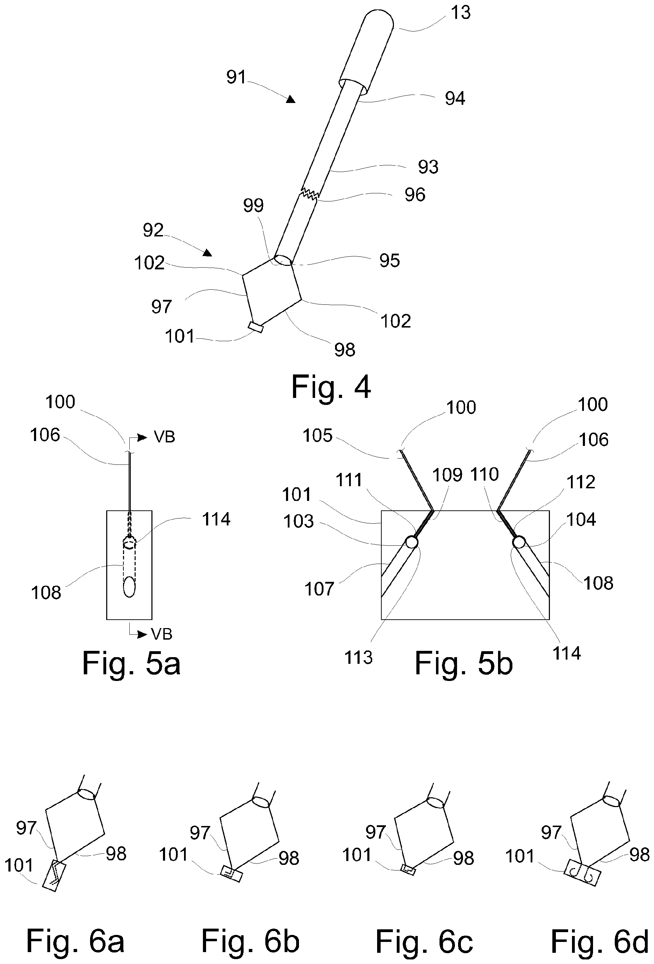

In fig. 4 a sketch of a second bipolar electrosurgical instrument 91 is for illustrative purposes shown fragmented by fragment lines 96. The second bipolar electrosurgical instrument 91 has all the features of the bipolar electrosurgical instrument 1 as described above. The only difference is how the electrodes 92 are elaborated. Like the bipolar electrosurgical instrument 1 the second bipolar electrosurgical instrument 91 has a hollow shaft 93 with a proximal end 94 and a distal end 95.

The electrodes of the second bipolar electrosurgical instrument 91 comprise two metal wire parts 97,98 that protrude from a first opening 99 in the distal end 95. The two metal wire parts 97, 98 are connected to the two electric conductors B21 and B22 from the circuit 9; 40. To avoid the two metal wire parts 97, 98 to touch each other and cause a short-circuit the distal end of the two metal wire parts 97, 98 are moulded into a holding member 101, where the two metal wire parts 97, 98 are not contacting each other. The holding member 101 is made of an insulating material. The advantage of this embodiment is that the two metal wire parts 97, 98 will never contact each other and cause a short-circuit.

The two metal wire parts 97, 98 both have a knee 102 so that the two metal wire parts 97, 98 are bent away from each other to avoid contacting each other and to allow an easy access of the tissue to be cut off between the two metal wire parts 97, 98.

To have the ends of the two metal wire parts 97, 98 to stay in the holding member 101, the ends of the two metal wire parts 97, 98 can have each their knot 103, 104 at the ends of the two metal wire parts that are in the holding member 101, as indicated in figs. 5a and 5b.

Fig. 5b shows a sectional view taken along line Vb-Vb in fig. 5a of the holding member 101 to illustrate the interior components of said holding member. First and second metal wire parts 105, 106 with first and a second ends, where the first end has the knot 103, 104 are introduced into and through a first channel 107, 108 and then through a second channel 109, 110 with the second end first. Since the diameter of the knot is smaller than or equally big as the first channel 107, 108 and is bigger than the second channel 109, 110 the knot will be caught at a second opening 111, 112 of the second channel 109, 110. When the first and second metal wire parts are introduced so far that the knot is pressed against the second opening 111, 112 of the second channel 109, 110 the first and second metal wire part 105, 106 are bent manually just outside the holding member 101. In that way the first and second metal wire parts are fixed. Where the first channel meets the second channel the first channel can have an increased diameter like a spherical cavity 113, 114. The advantage of the spherical cavity is that the knot will be prevented to fall back through the first channel 107, 108. The knot can be made of an elastic material and the first channel can be made just a little smaller than the diameter of the knot so that the knot has to be squeezed when pulled through the first channel. The ends of the first and second metal wire part 105, 106 sticking out of the holding member 101 are welded or in any other way attached to the two metal wire parts 97, 98 or to the two electric conductors B21 and B22. In figs. 5a and 5b the first and second wire parts 105, 106 is for illustrative purposes shown fragmented by fragment lines 100.

The two metal wire parts 97, 98 can also be bent as shown in figs. 6a - d. The advantage is here that the two second metal wire parts 105, 106 do not need to be welded or in any other way attached to the metal wire parts 97, 98. The metal wire parts can just be moulded or glued into the holding member 101.

It is to be understood that within the scope of the present invention the features, such as various circuits, can be combined as desired and feasible, into embodiments and modified instruments not shown in the figures. Thus the embodiments shown in said figures should not be construed as limiting the scope of the present invention: E.g. the embodiment of an electrosurgical instrument 1 and 91, shown in fig. 1 as well as fig. 4 (including the embodiments of the loop electrode shown in figs. 5a, 5b and 6a - 6d), may also comprise any of the circuits shown in figs. 2a - 2b and 3a - 3d.

The present invention proposes an inexpensive bipolar electrosurgical instrument for use with most conventional bipolar electrosurgical generators as well as with bipolar electrosurgical generators specifically designed for use with said instrument.

## Claims

1. An electrosurgical instrument (1) comprising a circuit (9; 40), a measurement unit (28; 44), means to be connected to an electrosurgical generator (2), and at least two electrodes (12, 16a, 16b, 16c, 16d; 97, 98) for cutting or coagulation by an RF-frequency signal from the electrosurgical generator, where the circuit (9; 40) has means to switch the electrosurgical instrument (1) between a measurement mode, wherein the measurement unit (28; 44) measures the impedance between the electrodes (12, 16a, 16b, 16c, 16d; 97, 98), and an operating mode, wherein the electrosurgical generator (2) applies a voltage over the electrodes (12, 16a, 16b, 16c, 16d; 97, 98), **characterised in that** the circuit (9; 40) comprises a timer (28; 44) configured to periodically control the switching of the electrosurgical instrument (1) between the measurement mode and the operating mode and **in that** the measurement unit is configured to measure the impedance between the electrodes by applying a DC signal.

2. An instrument (1) according to claim 1, **characterised in that** the circuit (9;40) has at least one first switch (25, 26, 27; 52, 53, 54) and at least one second switch (25, 26, 27; 52, 53, 54), controlled by the circuit (9; 40), where, in the measurement mode, the at least one first switch (25, 26, 27; 52, 53, 54) is in a mode so that the electrosurgical generator (2) is disconnected from the electrodes (12, 16a, 16b, 16c, 16d; 97, 98) and the at least one second switch (25, 26, 27; 52, 53, 54) is in a mode so that the measurement unit (28; 44) is connected to the electrodes (12, 16a, 16b, 16c, 16d; 97, 98).

3. An instrument (1) according to any of claims 1 or 2, **characterised in that** the circuit (9; 40) has at least one third switch (25, 26, 27; 52, 53, 54) and at least one fourth switch (25, 26, 27; 52, 53, 54), controlled by the circuit (9; 40), where, in the operating mode, the at least one third switch (25, 26, 27; 52, 53, 54) is in a mode so that the electrosurgical generator (2) is connected to the electrodes (12, 16a, 16b, 16c, 16d; 97, 98) and the at least one fourth switch (25, 26, 27; 52, 53, 54) is in a mode so that the measurement unit (28; 44) is disconnected from the electrodes (12, 16a, 16b, 16c, 16d; 97, 98).

4. An instrument (1) according to any of the preceding claims, **characterised in that** the measurement unit (28; 44) has at least one of the following means or combinations thereof:
- means to apply a voltage over the electrodes (12, 16a, 16b, 16c, 16d; 97, 98),
- means to measure the current through the electrodes (12, 16a, 16b, 16c, 16d; 97, 98),
- means to calculate the impedance of an object between the electrodes (12, 16a, 16b, 16c, 16d; 97, 98) and
- means to, if the impedance is below a first predefined limit or above a second predefined limit, stop the circuit (9; 40) to switch over the electrosurgical instrument (1) to the operating mode.

5. **An instrument according to any of the preceding claims, characterised in that** the timer (28; 44) is configured to periodically control the switching of the electrosurgical instrument (1) between the measurement mode and the operating mode with a period that is preferably between 0.05 and 0.15 of the period in the measurement mode and 0.85 and 0.95 of the period in the operating mode and most preferably one tenth of the period in the measurement mode and nine tenth of the period in the operating mode.

6. An instrument according to any of the preceding claims, **characterised in that** the timer (28; 44) is configured to periodically control the switching of the electrosurgical instrument (1) between the measurement mode and the operating mode with a period that is less than 5 sec. long.

7. An instrument according to any of the preceding claims, comprising at least one first switch (25, 26, 27; 52, 53, 54), controlled by the circuit (9; 40) to disconnect the electrosurgical generator (2) from the electrodes (12, 16a, 16b, 16c, 16d; 97, 98) and to connect the measurement unit (28; 44) to the electrodes (12, 16a, 16b, 16c, 16d; 97, 98) when going from the operating mode to the measurement mode,.

8. An instrument according to any of the preceding claims, comprising at least one second switch (25, 26, 27; 52, 53, 54), controlled by the circuit (9; 40), to disconnect the measurement unit (28; 44) from the electrodes (12, 16a, 16b, 16c, 16d; 97, 98) and to connect the electrosurgical generator (2) to the electrodes (12, 16a, 16b, 16c, 16d; 97, 98) when going from the measurement mode to the operating mode,.

9. An instrument according to any of the preceding claims, **characterised in that** the measurement unit (28; 44) in the measurement mode is configured to apply a voltage over the electrodes (12, 16a, 16b, 16c, 16d; 97, 98), measure the current through the electrodes (12, 16a, 16b, 16c, 16d; 97, 98), calculate the impedance of an object between the electrodes (12, 16a, 16b, 16c, 16d; 97, 98) and if the impedance is below a first predefined limit or above a second predefined limit, the circuit (9; 40) will not switch over to the operating mode.

10. An instrument according to any of the preceding claims, **characterised in that** the measurement unit (28; 44) is configured to calculate a cutting voltage or a coagulating voltage based on the impedance, and that the measurement unit (28; 44) is configured to control the electrosurgical generator (2) to apply the calculated cutting voltage or coagulating voltage in the operating mode.

## Patentansprüche

1. Elektrochirurgisches Instrument (1), das eine Schaltung (9; 40), eine Messeinheit (28; 44), Mittel, die mit einem elektrochirurgischen Generator (2) verbunden werden, und mindestens zwei Elektroden (12, 16a, 16b, 16c, 16d; 97, 98) zum Schneiden oder Koagulieren durch ein HF-Frequenzsignal vom elektrochirurgischen Generator umfasst, wobei die Schaltung (9; 40) Mittel zum Umschalten des elektrochirurgischen Instruments (1) zwischen einem Messmodus, wobei die Messeinheit (28; 44) die Impedanz zwischen den Elektroden (12, 16a, 16b, 16c, 16d; 97, 98) misst, und einem Betriebsmodus aufweist, wobei der elektrochirurgische Generator (2) eine Spannung an die Elektroden (12, 16a, 16b, 16c, 16d; 97, 98) anlegt, **dadurch gekennzeichnet, dass** die Schaltung (9; 40) einen Timer (28; 44) umfasst, der zur periodischen Kontrolle der Schaltung des elektrochirurgischen Instrumentes (1) zwischen dem Messmodus und dem Betriebsmodus konfiguriert ist, und dass die Messeinheit zur Messung der Impedanz zwischen den Elektroden durch Anlegen eines DC-Signals konfiguriert ist.

2. Instrument (1) nach Anspruch 1, **dadurch gekennzeichnet, dass** die Schaltung (9; 40) mindestens einen ersten Schalter (25, 26, 27; 52, 53, 54) und mindestens einen zweiten Schalter (25, 26, 27; 52, 53, 54) aufweist, gesteuert durch die Schaltung (9; 40), wobei sich im Messmodus der mindestens eine erste Schalter (25, 26, 27; 52, 53, 54) in einem derartigen Modus befindet, dass der elektrochirurgische Generator (2) von den Elektroden (12, 16a, 16b, 16c, 16d; 97, 98) getrennt ist, und der mindestens eine zweite Schalter (25, 26, 27; 52, 53, 54) sich in einem derartigen Modus befindet, dass die Messeinheit (28; 44) mit den Elektroden (12, 16a, 16b, 16c, 16d; 97, 98) verbunden ist.

3. Instrument (1) nach einem der Ansprüche 1 oder 2, **dadurch gekennzeichnet, dass** die Schaltung (9; 40) mindestens einen dritten Schalter (25, 26, 27; 52, 53, 54) und mindestens einen vierten Schalter (25, 26, 27; 52, 53, 54) aufweist, gesteuert von der Schaltung (9; 40), wobei sich im Betriebsmodus der mindestens eine dritte Schalter (25, 26, 27; 52, 53, 54) in einem derartigen Modus befindet, dass der elektrochirurgische Generator (2) mit den Elektroden (12, 16a, 16b, 16c, 16d; 97, 98) verbunden ist, und sich der mindestens eine vierte Schalter (25, 26, 27; 52, 53, 54) in einem derartigen Modus befindet, dass die Messeinheit (28; 44) von den Elektroden (12, 16a, 16b, 16c, 16d; 97, 98) getrennt ist.

4. Instrument (1) nach einem der vorhergehenden Ansprüche, **dadurch gekennzeichnet, dass** die Messeinheit (28; 44) mindestens eines der folgenden Mittel oder Kombinationen davon aufweist:
- Mittel zum Anlegen einer Spannung an die Elektroden (12, 16a, 16b, 16c, 16d; 97, 98),
- Mittel zum Messen des Stroms durch die Elektroden (12, 16a, 16b, 16c, 16d; 97, 98),
- Mittel zum Berechnen der Impedanz eines Objekts zwischen den Elektroden (12, 16a, 16b, 16c, 16d; 97, 98), und
- Mittel zum Stoppen der Schaltung (9; 40), um das elektrochirurgische Instrument (1) in den Betriebsmodus umzuschalten, wenn die Impedanz unter einer ersten vordefinierten Grenze oder oberhalb einer zweiten vordefinierten Grenze liegt.

5. Instrument nach einem der vorhergehenden Ansprüche, **dadurch gekennzeichnet, dass** der Timer (28; 44) dafür konfiguriert ist, die Schaltung des elektrochirurgischen Instruments (1) zwischen dem Messmodus und dem Betriebsmodus periodisch mit einem Zeitraum zu steuern, der bevorzugt zwischen 0,05 und 0,15 des Zeitraums im Messmodus, und 0,85 und 0,95 des Zeitraums im Betriebsmodus, und am meisten bevorzugt ein Zehntel des Zeitraums im Messmodus und neun Zehntel des Zeitraums im Betriebsmodus beträgt.

6. Instrument nach einem der vorhergehenden Ansprüche, **dadurch gekennzeichnet, dass** der Timer (28; 44) konfiguriert ist, um das Umschalten des elektrochirurgischen Instruments (1) zwischen dem Messmodus und dem Betriebsmodus mit einem Zeitraum, der kürzer als 5 Sekunden lang ist, periodisch zu steuern.

7. Instrument nach einem der vorhergehenden Ansprüche, umfassend mindestens einen ersten Schalter (25, 26, 27; 52, 53, 54), der von der Schaltung (9; 40) gesteuert wird, um den elektrochirurgischen Generator (2) von den Elektroden (12, 16a, 16b, 16c, 16d; 97, 98) zu trennen und zum Verbinden der Messeinheit (28; 44) mit den Elektroden (12, 16a, 16b, 16c, 16d; 97, 98) beim Verlassen des Betriebsmodus in den Messmodus.

8. Instrument nach einem der vorhergehenden Ansprüche, umfassend mindestens einen zweiten Schalter (25, 26, 27; 52, 53, 54), der von der Schaltung (9; 40) gesteuert wird, um die Messeinheit (28; 44) von den Elektroden (12, 16a, 16b, 16c, 16d; 97, 98) zu trennen und den elektrochirurgischen Generator (2) mit den Elektroden (12, 16a, 16b, 16c, 16d; 97, 98) zu verbinden, wenn vom Messmodus in den Betriebsmodus gewechselt wird.

9. Instrument nach einem der vorhergehenden Ansprüche, **dadurch gekennzeichnet, dass** die Messeinheit (28; 44) im Messmodus konfiguriert ist, um eine Spannung an die Elektroden (12, 16a, 16b, 16c, 16d; 97, 98) anzulegen, den Strom durch die Elektroden (12, 16a, 16b, 16c, 16d; 97, 98) zu messen, die Impedanz eines Objektes zwischen den Elektroden (12, 16a, 16b, 16c, 16d; 97, 98) zu berechnen, und wenn die Impedanz unter einer ersten vordefinierten Grenze oder über einer zweiten vordefinierten Grenze liegt, wird die Schaltung (9; 40) nicht zum Betriebsmodus umschalten.

10. Instrument nach einem der vorhergehenden Ansprüche, **dadurch gekennzeichnet, dass** die Messeinheit (28; 44) konfiguriert ist, um eine Schneidespannung oder eine Koagulierungsspannung auf Basis der Impedanz zu berechnen, und die Messeinheit (28; 44) konfiguriert ist, um den elektrochirurgischen Generator (2) derart zu steuern, dass die berechnete Schneidespannung oder Koagulierungsspannung im Betriebsmodus angewandt wird.

## Revendications

1. Instrument électro-chirurgical (1) comprenant un circuit (9 ; 40), une unité de mesure (28 ; 44), un moyen permettant d'être connecté à un générateur électro-chirurgical (2), et au moins deux électrodes (12, 16a, 16b, 16c, 16d ; 97, 98) permettant la coupure ou la coagulation au moyen d'un signal de fréquence RF à partir du générateur électro-chirurgical, où le circuit (9 ; 40) possède un moyen pour commuter l'instrument électro-chirurgical (1) entre un mode de mesure, dans lequel l'unité de mesure (28 ; 44) mesure l'impédance entre les électrodes (12, 16a, 16b, 16c, 16d ; 97, 98), et un mode de fonctionnement, dans lequel le générateur électro-chirurgical (2) applique une tension aux électrodes (12, 16a, 16b, 16c, 16d ; 97, 98), **caractérisé en ce que** le circuit (9 ; 40) comprend une minuterie (28 ; 44) conçue pour commander de manière périodique la commutation de l'instrument électro-chirurgical (1) entre le mode de mesure et le mode de fonctionnement et **en ce que** l'unité de mesure est conçue pour mesurer l'impédance entre les électrodes en appliquant un signal de courant continu.

2. Instrument (1) selon la revendication 1, **caractérisé en ce que** le circuit (9 ; 40) possède au moins un premier commutateur (25, 26, 27 ; 52, 53, 54) et au moins un deuxième commutateur (25, 26, 27 ;52, 53, 54), commandés par le circuit (9 ; 40), où, dans le mode de mesure, l'au moins un premier commutateur (25, 26, 27 ; 52, 53, 54) se trouve dans un mode de sorte que le générateur électro-chirurgical (2) est déconnecté des électrodes. (12, 16a, 16b, 16c, 16d ; 97, 98) et l'au moins un deuxième commutateur (25, 26, 27 ; 52, 53, 54) se trouve dans un mode de sorte que l'unité de mesure (28 ; 44) est connectée aux électrodes (12, 16a, 16b, 16c, 16d ; 97, 98).

3. Instrument (1) selon la revendication 1 ou la revendication 2, **caractérisé en ce que** le circuit (9 ; 40) possède au moins un troisième commutateur (25, 26, 27 ; 52, 53, 54) et au moins quatrième commutateur (25, 26, 27 ; 52, 53, 54), commandés par le circuit (9 ; 40), où, dans le mode de mesure, l'au moins un troisième commutateur (25, 26, 27 ; 52, 53, 54) se trouve dans un mode de sorte que le générateur électro-chirurgical (2) est connecté aux électrodes. (12, 16a, 16b, 16c, 16d ; 97, 98) et l'au moins un quatrième commutateur (25, 26, 27 ; 52, 53, 54) se trouve dans un mode de sorte que l'unité de mesure (28 ; 44) est déconnectée des électrodes (12, 16a, 16b, 16c, 16d ; 97, 98).

4. Instrument (1) selon l'une quelconque des revendications précédentes, **caractérisé en ce que** l'unité de mesure (28 ; 44) possède au moins l'un des moyens ou combinaisons de ceux-ci suivant(e)s :
- un moyen d'appliquer une tension aux électrodes (12, 16a, 16b, 16c, 16d ; 97, 98),
- un moyen de mesurer le courant grâce aux électrodes (12, 16a, 16b, 16c, 16d ; 97, 98),
- un moyen de calculer l'impédance d'un objet entre les électrodes (12, 16a, 16b, 16c, 16d ; 97, 98) et
- un moyen, si l'impédance est inférieure à une première limite prédéfinie ou supérieure à une seconde limite prédéfinie, de stopper le circuit (9 ; 40) de commuter l'instrument électro-chirurgical (1) dans le mode de de fonctionnement.

5. Instrument selon l'une quelconque des revendications précédentes, **caractérisé en ce que** la minuterie (28 ;44) est conçue pour commander de manière périodique la commutation de l'instrument électro-chirurgical (1) entre le mode de mesure et le mode de fonctionnement avec une période qui est de préférence comprise entre 0,05 et 0,15 de la période dans le mode de mesure et comprise entre 0,85 et 0,95 de la période dans le mode de fonctionnement et le plus préférablement 1/10 de la période dans le mode de mesure et 9/10 de la période dans le mode de fonctionnement.

6. Instrument selon l'une quelconque des revendications précédentes, **caractérisé en ce que** la minuterie (28 ;44) est conçue pour commander de manière périodique la commutation de l'instrument électro-chirurgical (1) entre le mode de mesure et le mode de fonctionnement avec une période qui est inférieure à 5 secondes.

7. Instrument selon l'une quelconque des revendications précédentes, comprenant au moins un premier commutateur (25, 26, 27 ; 52, 53, 54), commandé par le circuit (9 ; 40) afin de déconnecter le générateur électro-chirurgical (2) des électrodes (12, 16a, 16b, 16c, 16d ; 97, 98) et de connecter l'unité de mesure (28 ; 44) aux électrodes (12, 16a, 16b, 16c, 16d ; 97, 98) quand il commute du mode de fonctionnement au mode de mesure.

8. Instrument selon l'une quelconque des revendications précédentes, comprenant au moins un deuxième commutateur (25, 26, 27 ; 52, 53, 54), commandé par le circuit (9 ; 40) afin de déconnecter l'unité de mesure (28 ; 44) des électrodes (12, 16a, 16b, 16c, 16d ; 97, 98) et de connecter le générateur électro-chirurgical (2) aux électrodes (12, 16a, 16b, 16c, 16d ; 97, 98) quand il commute du mode de mesure au mode de fonctionnement.

9. Instrument selon l'une quelconque des revendications précédentes, **caractérisé en ce que** l'unité de mesure (28 ; 44) dans le mode de mesure est conçue pour appliquer une tension aux électrodes (12, 16a, 16b, 16c, 16d ; 97, 98), mesurer le courant grâce aux électrodes (12, 16a, 16b, 16c, 16d ; 97, 98), calculer l'impédance d'un objet entre les électrodes (12, 16a, 16b, 16c, 16d ; 97, 98) et si l'impédance est inférieure à une première limite prédéfinie ou supérieure à une seconde limite prédéfinie, le circuit (9 ; 40) ne commutera pas dans le mode de fonctionnement.

10. Instrument selon l'une quelconque des revendications précédentes, **caractérisé en ce que** l'unité de mesure (28 ; 44) est conçue pour calculer une tension de coupure ou une tension de coagulation en fonction de l'impédance, et **en ce que** l'unité de mesure (28 ; 44) est conçue pour commander le générateur électro-chirurgical (2) pour qu'il applique la tension de coupure ou la tension de coagulation calculée dans le mode de fonctionnement.
